## Europäisches Patentamt
## European Patent Office
## Office européen des brevets

(19)

(11) Numéro de publication: **0 054 041**
**B1**

(12) # FASCICULE DE BREVET EUROPEEN

(45) Date de publication du fascicule du brevet:
**16.05.84**

(51) Int. Cl.³: **A 41 B 13/02**

(21) Numéro de dépôt: **81901518.1**

(22) Date de dépôt: **02.06.81**

(86) Numéro de dépôt international:
**PCT/FR 81/00067**

(87) Numéro de publication internationale:
**WO 81/03601 (24.12.81 Gazette 81/30)**

(54) **ATTACHES ELASTIQUES POUR COUCHE-CULOTTE ET PROCEDE DE FABRICATION DE TELLES ATTACHES.**

(30) Priorité: **19.06.80 FR 8013577**

(43) Date de publication de la demande:
**23.06.82 Bulletin 82/25**

(45) Mention de la délivrance du brevet:
**16.05.84 Bulletin 84/20**

(84) Etats contractants désignés:
**AT CH DE GB LI LU NL SE**

(56) Documents cités:
**FR - A - 2 289 131**
**FR - A - 2 289 133**
**FR - A - 2 375 838**
**FR - A - 2 413 891**
**US - A - 3 869 761**

(73) Titulaire: **BEGHIN-SAY SOCIETE ANONYME,**
**F-59239 Thumeries (FR)**

(72) Inventeur: **LAPLANCHE, Pierre, 7, rue de Katzenthal,**
**F-68230 Turckheim (FR)**

(74) Mandataire: **Quéré, Jean Pierre, BEGHIN-SAY Service**
**Propriété Industrielle 54, Avenue Hoche, F-75008 Paris**
**(FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

Attaches élastique pour couche-culotte

### Domaine technique

La présente invention se rapporte à une attache élastique utilisable notamment comme système de fixation permettant de lier la partie avant et la partie arrière d'une couche-culotte à jeter pour les bébés.

Elle se rapporte également à un procédé de fabrication d'une telle attache sur une machine de production de couches-culottes à jeter.

Les couches-culottes à jeter pour bébés sont formées d'une masse absorbante, constituée d'un matelas de fibres cellulosiques, placée entre une feuille support imperméable et une feuille perméable aux liquides, dite feuille de non-tissé, destinée à être en contact avec la peau du bébé.

### Technique antérieure

Pour maintenir la couche-culotte autour de la taille du bébé, on utilise des attaches adhésives dont une extrémité est fixée sur la feuille support imperméable et l'autre extrémité, enduite d'un adhésif protégé par un papier siliconé, est libre. Au moment de l'utilisation de la couche-culotte, on enlève le papier siliconé et on applique la partie adhésive découverte sur la partie arrière supérieure de la feuille support imperméable.

Pour éviter une liaison trop rigide entre les parties avant et arrière de la couche-culotte, on a cherché à rendre les attaches élastiques.

Le brevet français No. 7 217 105 décrit une bande d'attache semi-élastique comportant une section centrale pouvant s'allonger librement et deux sections terminales non extensibles, une des sections terminales étant fixée à un bord latéral de la couche au niveau de la taille de l'enfant, l'autre section terminale étant libre et ayant une surface revêtue d'adhésif par pression.

On connaît également une attache élastique, décrite dans le brevet américain 3 920 018, constituée par une boucle extensible ou attache pliée en C.

Lors de la fabrication des couches-culottes, les attaches se trouvent en partie hors de la surface définie par les couches-culottes, ce qui rend impossible l'obtention de fortes cadences de fabrication. De plus, dans le cas particulier de l'attache à boucle extensible, la nécessité d'insérer le doigt dans la boucle mal formée complique le geste d'ouverture de l'attache.

Pour ces raisons, on a recherché une structure nouvelle d'attache qui offre d'une part une bonne élasticité lors de la fixation de la couche-culotte autour de la taille du bébé et d'autre part, une certaine rigidité d'ensemble permettant de maintenir l'attache dans la surface des couches-culottes lors de leur fabrication, et de façon générale avant utilisation de l'attache.

Le brevet français No. 7 800 341 au nom de la demanderesse, décrit une attache élastique pliée en S, caractérisée en ce qu'elle comporte une première zone non élastique, une zone centrale élastique, et une seconde zone non élastique, dont la face en regard de la zone centrale élastique est revêtue d'un adhésif sensible à la pression, recouvert d'un élément protecteur, lui-même lié à la zone centrale par une ligne de colle continue ou discontinue parallèle aux lignes de pliage de l'attache.

Lors de l'utilisation de cette attache, on tire l'extrémité libre de la seconde zone, l'élément protecteur se décolle de cette seconde zone et reste fixé à la zone élastique. Dès lors, on peut fixer la seconde zone sur le bord d'une couche-culotte.

Toutefois, cette attache présente l'inconvénient d'être constituée en trois éléments distincts (une partie centrale et deux parties terminales) qui sont ensuite assemblés au moyen d'adhésifs.

La fabrication d'une telle attache se heurte à des difficultés lorsqu'elle doit être entreprise sur une machine de production de couches-culottes à fort rendement.

D'autre part, l'élément protecteur de la partie libre de l'attache est relié à l'une des faces de la partie médiane élastique au moyen d'une ligne de colle.

Dans la pratique, cette liaison s'est avérée difficile à réaliser.

### Exposé de l'invention

Le but de la présente invention est de réaliser une attache élastique qui présente aussi une certaine rigidité jusqu'au moment de l'utilisation seulement, mais dont la structure simplifiée rende possible une fabrication à grand rendement sur la machine de production des couches-culottes.

Pour parvenir à ce but, les deux parties latérales de l'attache (non élastiques) sont constituées d'une seule pièce par une bande support munie d'une ligne de prédécoupe (ligne de perforations), lesdites parties latérales étant au moment de l'utilisation séparées au niveau de cette prédécoupe par traction sur la partie latérale libre; après cette rupture, les deux parties latérales restent liées ensemble élastiquement par la partie centrale extensible fixée à l'une et à l'autre des parties latérales.

La partie centrale extensible peut être reliée à l'une et à l'autre des parties latérales au moyen de deux bandes d'adhésif disposées sur une même première face de la bande support de part et d'autre de la ligne de prédécoupe.

La seconde face de la bande support est revêtue de deux bandes d'adhésif pour le maintien de l'une et l'autre parties terminales de l'attache sur les faces respectivement avant et arrière de la couche-culotte.

De façon préférentielle, cette seconde face de la bande support comporte, en outre, une bande d'adhésif médiane recouvrant la position de la ligne de prédécoupe.

De préférence également, des bandes d'adhésif recouvrant l'une et l'autre faces de la bande support sont disposées en »quinconce«: en aucun point de la bande support, celle-ci n'est adhésivée sur les deux faces.

Selon un premier mode de réalisation de l'invention, la ligne de prédécoupe partage la bande support en deux parties d'égale longueur, et l'attache est pliée en deux selon une ligne de pliage confondue avec la ligne de prédécoupe.

Selon une première variante d'exécution, la partie latérale libre est plus longue que la partie latérale fixe, et l'attache est pliée en deux selon une ligne de pliage située dans la partie latérale libre non recouverte par la partie centrale extensible.

Selon une seconde variante d'exécution, il est prévu une partie supplémentaire dont une première face joue le rôle de bande protectrice de la partie latérale libre et la seconde face, adhésivée, permet une fixation supplémentaire sur le bord de la couche. Cette partie supplémentaire, de préférence fixée à la face externe du bord de la couche, permet de répartir la force de traction à la fois sur la face intérieure et la face extérieure du bord de la couche sur lequel l'attache est fixée.

Le procédé de fabrication de l'attache peut être réalisé de manière simple à très forte cadence à partir d'un rouleau de bandes supports de largeur égale à la longueur souhaitée des attaches.

Pour réaliser cette attache à partir d'un rouleau de bandes supports déjà recouvert sur ses deux faces des bandes adhésives indiquées ci-dessus, il suffit de réaliser la ligne de prédécoupe par tout moyen connu adéquat, de poser longitudinalement la bande extensible (sens travers) sur les bandes adhésives de la première face du rouleau de bandes supports, de plier la structure obtenue selon la ligne de pliage choisie, de poser ensuite la bande protectrice (papier siliconé), puis de découper la structure obtenue en une série d'attaches, qui sont ensuite fixées sur les couches-culottes.

Les différentes variantes d'exécution de l'attache peuvent être fabriquées en modifiant légèrement certaines étapes de ce procédé, comme on le verra plus loin dans la suite du texte de la description de plusieurs exemples de réalisation non limitatifs de l'objet de l'invention accompagné de dessins dans lesquels:

la fig. 1 est une vue en coupe de l'attache à plat selon l'invention,

la fig. 2 est une vue en coupe de l'attache de la fig. 1, pliée et posée sur une couche-culotte avant utilisation,

la fig. 3 est une vue en coupe de l'attache des fig. 1 et 2, en position d'utilisation,

la fig. 4 est une vue en coupe de l'attache à plat selon une première variante de réalisation de l'invention,

la fig. 5 est une vue en coupe de l'attache de la fig. 3, pliée et posée sur une couche-culotte,

la fig. 6 est une vue en coupe de l'attache à plat selon une deuxième variante de réalisation de l'invention,

la fig. 7 est une vue en coupe de l'attache de la fig. 6, pliée et posée sur une couche-culotte,

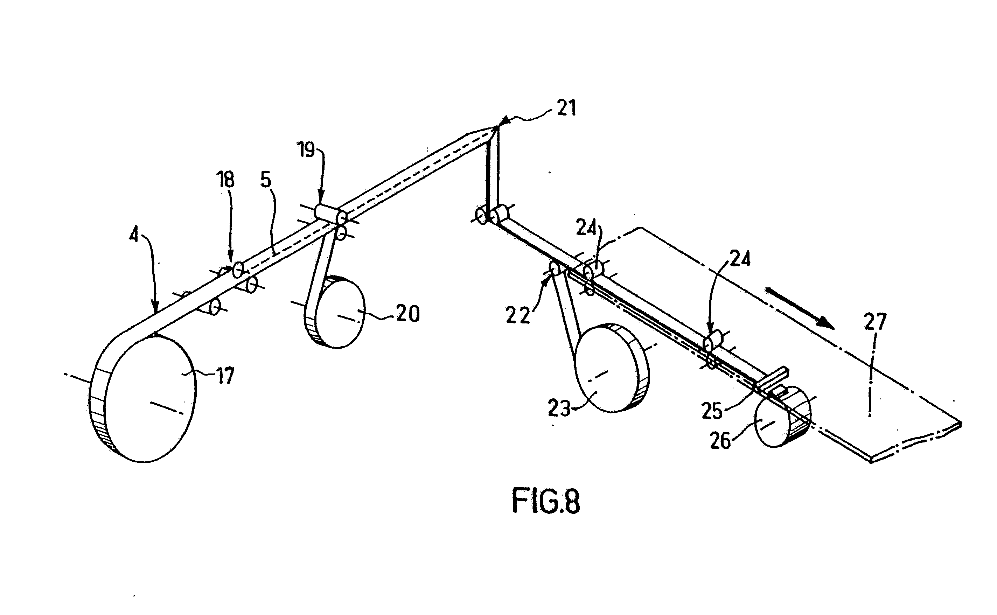

la fig. 8 est un schéma représentant les diverses étapes du procédé de réalisation d'une attache.

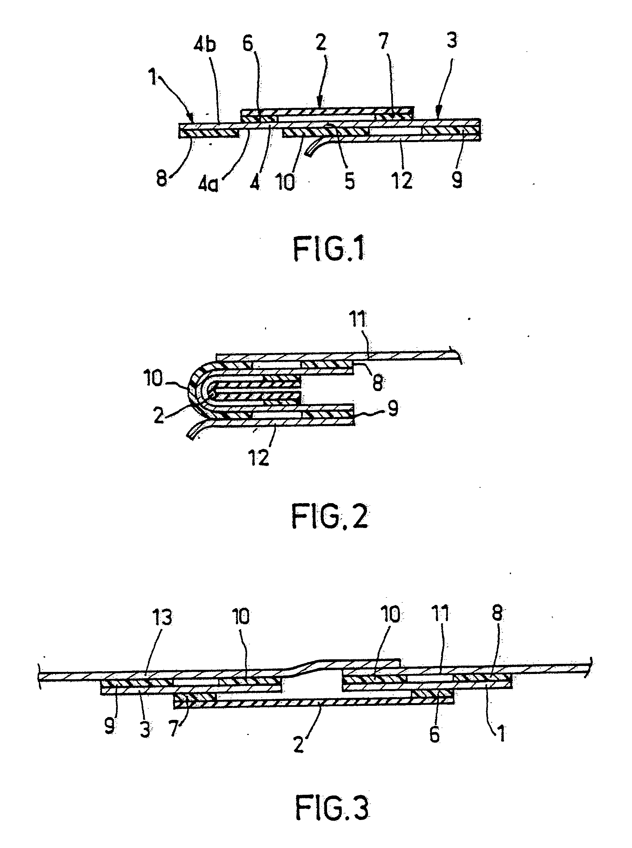

Sur le dessin des fig. 1, 2 et 3, on a représenté un premier exemple de réalisation de l'invention. L'attache comporte trois parties: une première partie latérale 1, une partie centrale extensible 2, (dans le sens de la longueur de l'attache) et une seconde partie latérale 3.

Les deux parties latérales 1 et 3 sont constituées en une seule pièce par une bande support 4, partagée en deux par une ligne de prédécoupe 5 (ligne de perforations par exemple), qui s'étend sur toute la largeur de la bande support.

La bande support 4 peut être en papier ou en matière plastique.

Cette bande support est revêtue d'un adhésif sur ses deux faces 4a et 4b. L'adhésif est déposé selon des bandes parallèles à la ligne de prédécoupe 5.

Sur la face 4b sont déposées deux bandes d'adhésif 6 et 7, disposées de part et d'autre de la ligne de prédécoupe 5, de largeur égale. Ces deux bandes d'adhésif permettent la fixation de la partie centrale extensible 2 de l'attache constituée par une laminette, un latex, un caoutchouc synthétique, un tissu élastique, ou tout autre matériau élastique équivalent.

La face 4a de la bande support 4 est revêtue de trois bandes d'adhésif 8, 9 et 10, qui sont disposées préférentiellement, comme on peut le voir sur la fig. 1, en »quinconce« avec les bandes d'adhésif 6 et 7 de la face 4b.

Les deux bandes d'adhésif 8 et 9 sont disposées aux extrémités de la bande support 4, pour servir de fixation des parties latérales de l'attache 1 et 3, respectivement sur l'une et l'autre des faces arrière et avant de la couche-culotte. De plus, il est prévu une bande d'adhésif médiane 10 recouvrant la position de la ligne de prédécoupe et s'étendant de part et d'autre de cette ligne sur l'une et l'autre des parties latérales 1 et 3. Les bandes d'adhésif 8, 9 et 10 recouvrant la face 4a s'étendent sur des zones de la bande support non recouvertes par les bandes d'adhésif 6 et 7 de la face 4b; en aucun point de la bande support, celle-ci n'est recouverte sur ses deux faces.

Comme on peut le voir sur la fig. 2 représentant l'attache pliée et fixée sur la couche-culotte avant utilisation, la bande d'adhésif 8 et la partie de la bande médiane située sur la partie latérale 1 servent à la fixation de l'attache sur la couche-culotte non représentée, mais seulement indiquée par la feuille 11, partie de la face supérieure arrière de la couche-culotte.

Une bande protectrice 12 (papier siliconé) recouvre la bande d'adhésif 9 et la partie de la bande médiane 10 située sur la partie latérale 3. Cette bande protectrice évite tout contact avec l'adhésif de la partie latérale libre de l'attache avant utilisation de celle-ci.

Une fois l'attache pliée et fixée sur la couche-culotte (voir fig. 2), l'attache est utilisée comme suit: l'utilisatrice saisit le bord de la bande protectrice 12 recouvrant la partie latérale libre 3 et l'attache, exerce une traction sur l'ensemble de cette partie latérale libre 3 suffisante pour rompre la bande support 4 au niveau de la ligne de prédécoupe 5. A ce moment, les deux parties latérales, bien que séparées, sont reliées élastiquement par la partie centrale extensible 2.

L'attache, rigide jusqu'au moment de l'utilisation, devient par la rupture de la ligne de prédécoupe une attache élastique, qui offre une possibilité d'adaptation de la fixation autour de la taille du bébé.

Sur la fig. 3, on a représenté l'attache en position d'utilisation, servant à la fixation des parties arrière et avant de la couche-culotte, non représentées mais seulement indiquées par leurs extrémités, référencées 11, respectivement 13.

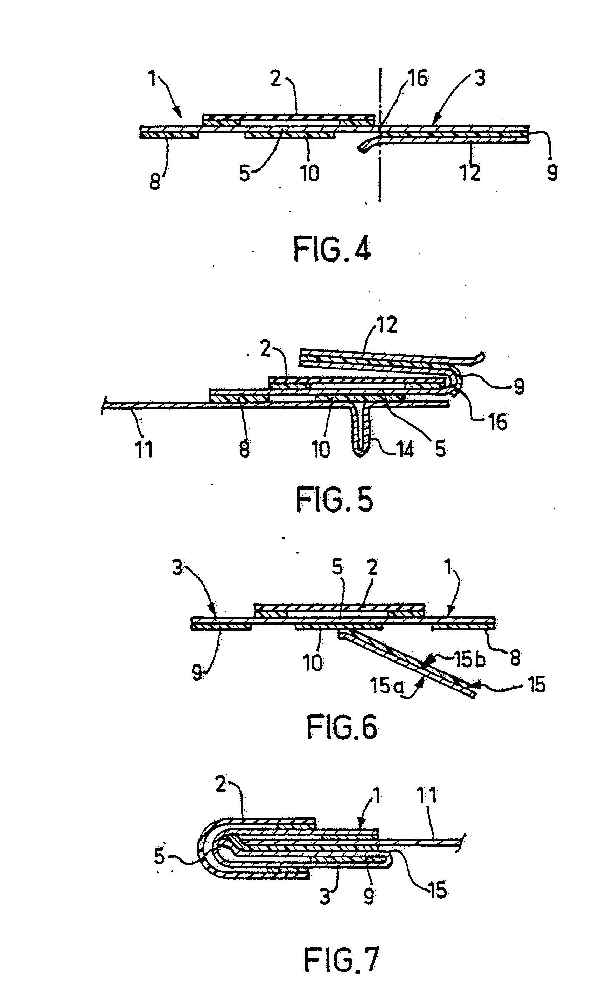

Dans l'exemple représenté sur les fig. 4 et 5, la ligne de prédécoupe 5 n'est plus située sensiblement au milieu de la longueur de l'attache. Contrairement au mode de réalisation décrit précédemment, la partie latérale libre 3 est plus longue que la partie latérale fixe 1.

La bande protectrice 12 ne recouvre que la bande d'adhésif 9, plus large que dans le mode de réalisation précédent.

Toutefois, c'est surtout dans le pliage et la pose de l'attache que cette variante se distingue du premier mode de réalisation.

Comme on peut le voir sur la fig. 5, l'attache est pliée, non plus autour d'une ligne confondue avec la ligne de prédécoupe, mais autour d'une ligne référencée 16 située sur la partie latérale libre 3 hors de la zone de cette partie latérale recouverte par la bande extensible 2.

De façon à permettre l'éloignement de l'une des parties latérales par rapport à l'autre au moment de l'utilisation, il est prévu sur la partie 11 de la couche sur laquelle l'attache est fixée un pli 14, qui est absorbé en tout ou partie avec l'allongement de la partie extensible 2.

Une seconde variante d'exécution est représentée sur le dessin des fig. 6 et 7.

L'attache comporte, outre les éléments déjà décrits dans le premier mode de réalisation, une partie supplémentaire 15 fixée par une de ses extrémités dans le voisinage de la ligne de prédécoupe 5, du côte de la partie latérale fixe 1.

Cette partie supplémentaire 15 comporte une première face 15a, siliconée pour servir de protecteur à l'adhésif de la partie latérale libre 3, et une face adhésivée 15b, destinée à être fixée, comme la partie latérale fixe, sur le bord 11 de la couche-culotte.

L'attache est pliée au niveau de la ligne de prédécoupe 5, et posée selon le dessin de la fig. 7.

Le bord 11 de la couche est »pincé« entre la partie latérale fixe 1 et la partie supplémentaire 15.

Comme ces deux parties 1 et 15 sont reliées ensemble au voisinage de la ligne de prédécoupe (voir fig. 6), la force de traction exercée sur l'attache lors de l'utilisation est répartie pour moitié sur l'une des faces du bord 11 de la couche et pour moitié sur l'autre face.

Une telle structure évite le glissement de l'attache sur le bord de la couche.

Pour réaliser l'attache des fig. 1 à 3, on part d'un rouleau de papier ou de matière plastique de largeur égale à la longueur des attaches, déjà revêtu d'un adhésif acrylique selon les bandes 6 à 10 décrites ci-dessus.

A titre d'exemple, on a fixé les dimensions suivantes:

— longueur de l'attache (largeur du rouleau de bande support)    70 mm
— largeur de l'attache    30 mm

| bande | largeur des bandes d'adhésif |
|-------|------------------------------|
| 6 | 8 mm |
| 7 | 8 mm |
| 8 | 13 mm |
| 9 | 20 mm |
| 10 | 13 mm |

— distance entre les bandes 6 et 7    24 mm

Les bandes supports, ainsi adhésivées, peuvent aussi être fournies sous forme de rouleaux 17 de plusieurs centaines de mètres, et montées sur le tambour d'alimentation du dispositif de fabrication des attaches, sur la machine de production des couches-culottes.

La bande support 4 est ensuite prédécoupée en 18 au moyen d'un outil de prédécoupe ou de perforation de type courant, de manière à réaliser la ligne de prédécoupe 5 des attaches. La profondeur des perforations et la distance choisie entre deux perforations, ou bien la profondeur de la ligne de prédécoupe, si celle-ci est continue, sont calculées de manière à offrir une bonne »déchirabilité« de la bande support lors de l'utilisation.

La partie médiane extensible 2 est ensuite posée sur les bandes adhésives 6 et 7 de l'une des faces du rouleau de bandes supports. Cet assemblage est réalisé en 19 au moyen de simples rouleaux presseurs alimentés d'une part par le rouleau de bandes supports, et d'autre part par un rouleau de laminette 20.

L'ensemble est ensuite plié en 21 autour de la ligne de prédécoupe selon une technique courante.

Sur la face correspondant à la partie terminale libre de la bande support ainsi repliée est fixée en 22 une bande protectrice siliconée délivrée par un rouleau 23.

La structure obtenue est ensuite engagée entre des rouleaux fournisseurs 24 qui la tendent, lui confèrent une vitesse très régulière et évitent tout glissement.

Un contre-couteau 25 tronçonne à intervalles réguliers la structure, de manière à obtenir les attaches individuelles, qui sont ensuite aspirées sur un couteau aspirant 26 avant d'être, après une fraction de tour sur ce couteau aspirant, projetées sur le complexe de couches-culottes 27 en des points correspondants aux zones de fixation des attaches.

Le procédé de réalisation des attaches selon la variante des fig. 4 et 5 est identique en tous points à celui décrit ci-dessus, excepté la position transversale de la ligne de prédécoupe et de la ligne de pliage.

A titre d'exemple, cette variante peut être illustrée par les dimensions suivantes:

| | | |
|---|---|---|
| — | longueur de l'attache | 90 mm |
| — | longueur de la partie terminale 1 | 35 mm |
| — | longueur de la partie terminale 3 | 55 mm |
| — | largeur des bandes 6 et 7 | 8 mm |
| — | distance entre les bandes 6 et 7 | 24 mm |
| — | largeur de la bande 8 | 13 mm |
| — | largeur de la bande 10 | 20 mm |
| — | largeur de la bande 9 | 33 mm |

Le procédé de réalisation des attaches selon les fig. 6 et 7 est une variante du procédé décrit précédemment.

Pour réaliser l'attache décrite selon les fig. 6 et 7, qui comprend une partie supplémentaire 15 de fixation, on supprime l'étape de pliage, et l'opération de pose de bande protectrice est remplacée par l'opération d'assemblage de cette partie supplémentaire sur le rouleau de bandes supports.

L'attache est fabriquée et posée à plat sur le complexe de couches-culottes 27.
C'est seulement lorsque l'attache est posée sur le complexe, qu'elle est pliée autour du bord de la couche-culotte.

**Revendications**

1. Attache élastique utilisable notamment pour la fixation des faces avant et arrière d'une couche-culotte pour bébés, comprenant trois parties; à savoir, une première partie latérale (1) fixée sur une face de la couche-culotte, une partie centrale extensible (2), et une seconde partie latérale libre (3) destinée à être fixée au moment de l'utilisation sur l'autre face de la couche-culotte au moyen d'un adhésif sensible à la pression, caractérisée en ce qu'avant utilisation les deux parties latérales de l'attache (1, 3) font partie d'une seule pièce constituée par une bande support (4) munie d'une ligne de prédécoupe (5), lesdites parties latérales étant, lors de l'utilisation, séparées au niveau de cette prédécoupe (5) par traction sur ladite seconde partie latérale libre (3), tout en restant liées ensemble élastiquement par la partie centrale extensible (2) fixée à l'une et à l'autre desdites parties latérales (1, 3).

2. Attache élastique selon la revendication 1, caractérisée en ce que la partie centrale extensible (2) est liée aux deux parties latérales (1, 3) au moyen de deux bandes d'adhésif (6, 7) disposées sur une même première face de la bande support (4) de part et d'autre de la ligne de prédécoupe (5).

3. Attache élastique selon la revendication 2, caractérisée en ce que la seconde face de la bande support (4) est revêtue d'adhésif pour la fixation des deux parties latérales (1, 3) de l'attache respectivement sur l'une et l'autre des faces avant et arrière de la couche-culotte.

4. Attache selon la revendication 3, caractérisée en ce que ladite seconde face de la bande support comporte en outre une bande médiane (10) d'adhésif recouvrant la ligne de prédécoupe (5) et s'étendant sur l'une et l'autre des parties latérales (1, 3) de l'attache.

5. Attache selon la revendication 4, caractérisée en ce que les bandes d'adhésif pour la fixation de la partie centrale extensible (2) sont disposées en »quinconce« de part et d'autre de la bande support (4).

6. Attache selon l'une quelconque des revendications 1 à 5, caractérisée en ce que la ligne de prédécoupe (5) est située sensiblement au milieu de la longueur de la bande support (4).

7. Attache selon la revendication 6, caractérisée en ce qu'une bande protectrice (12) recouvre à la fois la bande d'adhésif de la seconde partie latérale libre (3) et la zone de la bande médiane d'adhésif (10) située du côté de la ligne de prédécoupe (5) appartenant à ladite seconde partie latérale libre (3).

8. Attache selon l'une quelconque des revendications 1 à 7, caractérisée en ce qu'elle est pliée en deux selon une ligne de pliage sensiblement confondue avec la ligne de prédécoupe (5).

9. Attache selon l'une quelconque des revendications 1 à 5, caractérisée en ce que la ligne de prédécoupe (5) est située de telle manière que la seconde partie latérale (3) est plus longue que la première partie latérale.

10. Attache selon la revendication 9, caractérisée en ce qu'elle est pliée en deux selon une ligne de pliage distincte de la ligne de prédécoupe (5), parallèle à celle-ci, située dans la partie latérale libre non recouverte par la partie centrale extensible.

11. Attache selon l'une quelconque des revendications 1 à 5, caractérisée en ce qu'elle comprend en outre une partie supplémentaire, fixée par une de ses extrémités à la partie latérale fixe de la bande support au voisinage immédiat de la ligne de prédécoupe (5), et adhésivée sur sa face dirigée vers ladite partie latérale fixe.

12. Attache selon la revendication 11, caractérisée en ce que la face non adhésivée de ladite partie supplémentaire est traitée de manière à servir de surface protectrice de la bande d'adhésif de la partie latérale libre.

13. Attache selon l'une quelconque des revendications 11 ou 12, caractérisée en ce qu'elle est pliée en deux selon une ligne de pliage sensiblement confondue avec la ligne de prédécoupe (5), la partie latérale fixe et la partie supplémentaire étant fixées sur le même bord de la couche-culotte, respectivement sur les faces interne et externe dudit bord.

14. Procédé de fabrication en continu d'attaches extensibles conformes à l'une quelconque des revendications 1 à 5 à partir de bandes supports, de parties centrales extensibles et de bandes protectrices délivrées en rouleaux, caractérisé par les étapes suivantes, prises en combinaison:

a) on déroule une bande support (4) préadhésivé sur ses deux faces,

b) la bande support (4) est prédécoupée selon une ligne (5) parallèle à la direction d'avancement au moyen d'un outil de prédécoupe de type connu en soi,

c) on colle sur la première face de la bande support la bande de partie centrale extensible (2),

d) on plie sur elle-même ladite bande support (4) selon une ligne de pliage confondue avec la ligne de prédécoupe (5) ou une ligne parallèle à celle-ci, de manière à ce que la partie centrale extensible (2) soit située à l'intérieur du pli formé,

e) on découpe la structure ainsi obtenue selon d) en une série d'attaches, qui seront ensuite fixées sur les couches-culottes en cours de fabrication.

15. Procédé selon la revendication 14, caractérisé en ce que la ligne de prédécoupe (5) est la ligne longitudinale médiane de la bande support (4), et ce que la ligne de pliage est la ligne de prédécoupe (5).

16. Procédé selon la revendication 14, caractérisé en ce que la ligne de prédécoupe (5) partage la largeur de la bande support (4) en deux demi-bandes de largeur inégale, et en ce que la ligne de pliage est située dans la demi-bande de plus grande largeur, à une distance de la ligne de prédécoupe (5) telle que le pliage n'affecte pas la bande de parties centrales extensibles (2).

17. Procédé selon l'une quelconque des revendications 14 à 16, caractérisé en ce qu'après le pliage de la bande support (4), on associe à la structure ainsi obtenue une bande protectrice (12) sur la ou les bande(s) d'adhésif de la partie latérale libre de l'attache.

18. Procédé de fabrication en continu d'attaches extensibles conformes à l'une quelconque des revendications 11 à 13, à partir de bandes supports (4), de parties centrales extensibles (2) et de parties supplémentaires, tous ces éléments étant délivrés en rouleaux déjà adhésivés, caractérisé par les étapes suivantes, prises en combinaison:

a) on déroule la bande support préadhésivée sur ses deux faces,

b) la bande support est prédécoupé selon une ligne parallèle à la direction d'avancement au moyen d'un outil de prédécoupe de type connu en soi,

c) on colle sur la première face de la bande support la bande de parties centrales extensibles,

d) on colle sur la seconde face de la bande support les parties supplémentaires de fixation selon une étroite bande longitudinale de ce dernier,

e) on découpe la structure ainsi obtenue selon d) en une série d'attaches, qui sont ensuite fixées sur les couches-culottes en cours de fabrication.

## Patentansprüche

1. Elastisches Band zur Verwendung insbesondere für die Befestigung der Vorder- und Rückseiten einer Windelhose für Babies, bestehend aus drei Teilen, nämlich einem ersten Seitenteil (1), der auf einer Seite der Windelhose befestigt ist, einem dehnbaren Teil (2) und einem freien zweiten Seitenteil (3) zur Befestigung im Augenblick der Anwendung auf der anderen Seite der Windelhose mittels eines druckempfindlichen Klebstoffs, dadurch gekennzeichnet, daß vor Gebrauch die zwei Seitenteile des Bandes (1, 3) Teil eines einzigen von einem mit einer Vorschnittlinie (5) ausgestatteten Trägerband (4) gebildeten Stückes sind und beim Gebrauch durch Ausübung einer Zugkraft auf den genannten freien zweiten Seitenteil (3) auf der Ebene des Vorschnitts (5) getrennt, werden während sie durch den auf dem einen und anderen der genannten Seitenteile (1, 3) befestigten dehnbaren Mittelteil elastisch miteinander verbunden bleiben.

2. Elastisches Band nach Anspruch 1, dadurch gekennzeichnet, daß der dehnbare mittlere Teil (2) mittels zweier Klebstoffbänder (6, 7), die auf ein und derselben ersten Seite des Trägerbandes (4) auf beiden Seiten der Vorschnittlinie (5) angeordnet sind, mit den zwei Seitenteilen (1, 3) verbunden ist.

3. Elastisches Band nach Anspruch 2, dadurch gekennzeichnet, daß die zweite Seite des Trägerbandes (4) zur Befestigung der beiden Seitenteile (1, 3) des Bandes jeweils auf der einen und anderen der Vorder- und Rückseiten der Windelhose mit Klebstoff versehen ist.

4. Band nach Anspruch 3, dadurch gekennzeichnet, daß die genannte zweite Seite des Trägerbandes außerdem ein mittleres Klebstoffband (10) enthält, welches die Vorschnittlinie (5) bedeckt und sich auf dem einen und anderen der Seitenteile (1, 3) des Bandes erstreckt.

5. Band nach Anspruch 4, dadurch gekennzeichnet, daß die Klebstoffbänder zur Befestigung des mittleren dehnbaren Teils (2) im »Zickzack« auf beiden Seiten des Trägerbandes (4) liegen.

6. Band nach irgendeinem der Ansprüche 1 bis 5, dadurch gekennzeichnet, daß die Vorschnittlinie (5) im wesentlichen in der Mitte der Länge des Trägerbandes (4) liegt.

7. Band nach Anspruch 6, dadurch gekennzeichnet, daß ein Schutzband (12) gleichzeitig das Klebband des zweiten freien Seitenteils (3) und die Zone des mittleren Klebstoffbandes (10) auf der dem zweiten freien Seitenteil (3) zugehörigen Seite der Vorschnittlinie (5) bedeckt.

8. Band nach irgendeinem der Ansprüche 1 bis 7, dadurch gekennzeichnet, daß es auf einer Faltlinie im wesentlichen gleichlaufend mit der Vorschnittlinie (5) in zwei Teile gefaltet ist.

9. Band nach irgendeinem der Ansprüche 1 bis 5, dadurch gekennzeichnet, daß die Vorschnittlinie (5) so angeordnet ist, daß der zweite Seitenteil (3) länger ist als der erste Seitenteil.

10. Band nach Anspruch 9, dadurch gekennzeichnet, daß es auf einer anderen Faltlinie als der Vorschnittlinie (5) in zwei Teile gefaltet ist, die parallel zu dieser verläuft und in dem nicht von dem dehnbaren mittleren Teil bedeckten freien Seitenteil liegt.

11. Band nach irgendeinem der Ansprüche 1 bis 5, dadurch gekennzeichnet, daß es außerdem einen zusätzlichen Teil enthält, der durch eines seiner Enden an dem festen Seitenteil des Trägerbandes in unmittelbarer Nachbarschaft der Vorschnittlinie (5) befestigt und auf seiner dem festen Seitenteil zugewendeten Seite mit Klebstoff versehen ist.

12. Band nach Anspruch 11, dadurch gekennzeichnet, daß die nicht mit Klebstoff versehene Seite des genannten zusätzlichen Teils derart behandelt ist, daß sie als Schutzfläche des Klebbandes des freien Seitenteils dient.

13. Band nach irgendeinem der Ansprüche 11 oder 12, dadurch gekennzeichnet, daß es auf einer Faltlinie im wesentlichen gleichlaufend mit der Vorschnittlinie (5) in zwei Teile gefaltet ist, wobei der feste Seitenteil und der zusätzliche Teil auf derselben Kante der Windelhose jeweils auf der Innen- und Außenseite der genannten Kante befestigt sind.

14. Verfahren zur kontinuierlichen Herstellung von dehnbaren Bändern nach irgendeinem der Ansprüche 1 bis 5, ausgehend von Trägerbändern, dehnbaren mittleren Teilen und in Rollen herangeführten Schutzbändern, gekennzeichnet durch die Kombination der folgenden Schritte:

a) Abrollen eines im Vorhinein auf seinen beiden Seiten mit Klebstoff versehenen Trägerbandes,

b) Vorschneiden des Trägerbandes (4) längs einer Linie (5) parallel zur Vorschubrichtung mittels eines an sich bekannten Vorschneidwerkzeugs,

c) Aufkleben des Bandes des dehnbaren mittleren Teils (2) auf die erste Seite des Trägerbandes,

d) Falten des Trägerbandes (4) auf sich selbst nach einer Faltlinie gleichlaufend mit der Vorschnittlinie (5) oder einer Linie parallel zu dieser derart, daß der dehnbare Mittelteil (2) im Inneren der gebildeten Falte liegt,

e) Zuschneiden der gemäß d) erzielten Struktur in eine Reihe von Bändern, die anschließend im Verlaufe der Fabrikation auf den Windelhosen befestigt werden.

15. Verfahren nach Anspruch 14, dadurch gekennzeichnet, daß die Vorschnittlinie (5) die Längsmittellinie des Trägerbandes (4) ist und die Faltlinie die Vorschnittlinie ist.

16. Verfahren nach Anspruch 14, dadurch gekennzeichnet, daß die Vorschnittlinie (5) die Breite des Trägerbandes (4) in zwei Bandhälften ungleicher Breite teilt und die Faltlinie bei einer solchen Entfernung von der Vorschnittlinie (5) in der Bandhälfte größerer Breite liegt, daß die Faltung nicht das Band der dehnbaren Mittelteile (2) beeinträchtigt.

17. Verfahren nach irgendeinem der Ansprüche 14 bis 16, dadurch gekennzeichnet, daß nach der Faltung des Trägerbandes (4) mit der so erlangten Struktur ein Schutzband (12) auf dem (oder den) Klebstoffband (-bändern) des freien Seitenteils des Bandes verbunden wird.

18. Verfahren zur kontinuierlichen Herstellung dehnbarer Bänder entsprechend irgendeinem der Ansprüche 11 bis 13, ausgehend von Trägerbändern (4), dehnbaren Mittelteilen (2) und zusätzlichen Teilen, wobei alle diese Elemente in schon mit Klebstoff versehenen Rollen herbeigeführt werden, gekennzeichnet durch die Kombination der folgenden Schritte:

a) Abrollen des vorher auf beiden Seiten mit Klebstoff versehenen Trägerbandes,

b) Vorschneiden des Trägerbandes nach einer Linie parallel zur Vorschubrichtung mittels eines an sich bekannten Vorschneidwerkzeugs,

c) Aufkleben des Bandes der dehnbaren Mittelteile auf die erste Seite des Trägerbandes,

d) Aufkleben der zusätzlichen Befestigungsteile gemäß einem schmalen in Längsrichtung verlaufenden Band der letzteren auf die zweite Seite des Trägerbandes,

e) Zuschneiden der so gemäß d) erlangten Struktur in eine Reihe Bänder, die anschließend im Produktionsverlauf auf den Windelhosen befestigt werden.

## Claims

1. An electric fastener adapted to be used in particular for fastening the front and rear faces of diaper panties for babies, comprising three parts, namely: a first side part (1) fixed on one face of the diaper panties, an extensible central part (2), and a second free side part (3) intended to be fastened at the time of use to the other face of the diaper panties by means of a pressure sensitive adhesive, characterized in that, before use, the two side parts of the fastener (1, 3) form part of a single piece composed of a support band (4) provided with a precut line (5), the said side parts being separated, at the time of use, along this precut line by pulling on the said second free side part (3), while nevertheless remaining joined together elastically by the extensible central part (2) fastened to each of the said side parts (1, 3).

2. An elastic fastener as claimed in claim 1, characterized in that the extensible central part (2) ist joined to the two side parts (1, 3) by means of two bands of adhesive (6, 7) disposed on one and the same first face of the support band (4) on each side of the precut line (5).

3. An elastic fastener as claimed in claim 2, characterized in that the second face of the support band (4) is coated with adhesive for the fastening of the two side parts (1, 3) of the fastener to the front and rear faces of the diaper panties respectively.

4. A fastener as claimed in claim 3, characterized in that the said second face of the support band comprises in addition a central band (10) of adhesive covering the precut line (5) and extending over both the side parts (1, 3) of the fastener.

5. A fastener as claimed in claim 4, characterized in that the bands of adhesive for fastening the extensible central part (2) are disposed in a staggered arrangement on each side of the support band (4).

6. A fastener as claimed in any of claims 1 to 5, characterized in that the precut line (5) is situated substantially in the middle of the length of the support band (4).

7. A fastener as claimed in claim 6, characterized in that a protective band (12) covers both the band of adhesive of the second free side part (3) and the zone of the central band of adhesive (10) situated on that side of the precut line (5) which belongs to the said second free side part (3).

8. A fastener as claimed in any one of claims 1 to 7, characterized in that it is folded in two along a fold line substantially coinciding with the precut line (5).

9. A fastener as claimed in any of claims 1 to 5, characterized in that the precut line (5) is situated in such a manner that the second side part (3) is longer than the first side part.

10. A fastener as claimed in claim 9, characterized in that it is folded in two along a fold line different from the precut line (5), parallel to the latter and situated in the free side part not covered by the extensible central part.

11. A fastener as claimed in any of claims 1 to 5, characterized in that it comprises also an additional part fastened by one of its ends to the fixed side part of the support band in the immediate proximity of the precut line (5) and coated with adhesive on its face directed towards the said fixed side part.

12. A fastener as claimed in claim 11, characterized in that the non-adhesive coated face of the said additional part is treated in such a manner as to serve as protective surface for the band of adhesive on the free side part.

13. A fastener as claimed in either of claims 11 or 12, characterized in that it is folded in two along a fold line substantially coinciding with the precut line (5), the fixed side part and the additional part being fastened on the same edge of the diaper panties, respectively on the inner and outer faces of the said edge.

14. A process for the continuous production of extensible fasteners according to any one of claims 1 to 5 from support bands, extensible central parts, and protective bands supplied in rolls, characterized by the following stages, taken in combination:

a) a support band (4) precoated with adhesive on both faces is unwound,

b) the support band (4) is precut along a line (5) parallel to the direction of advance with the aid of a precutting tool of a type known per se,

c) the extensible central part band (2) is bonded to the first face of the support band,

d) the said support band (4) is folded over itself along a fold line coinciding with the precut line (5) or along a line parallel thereto, in such a manner that the extensible central part (2) is situated inside the resulting fold,

e) the structure thus obtained in accordance with d) is cut up into a series of fasteners which will then be fixed on the diaper panties in the course of manufacture.

15. A process as claimed in claim 14, characterized in that the precut line (5) is the longitudinal median line of the support band (4), and that the fold line is the precut line (5).

16. A process as claimed in claim 14, characterized in that the precut line (5) divides the width of the support band (4) into two half-bands of unequal widths, and in that the fold line is situated in the wider half-band, at a distance from the precut line (5) such that the fold does not affect the band of extensible central parts (2).

17. A process as claimed in any one of claims 14 to 16, characterized in that after the folding of the support band (4) the resulting structure is provided with a protective band (12) on the band or bands of adhesive on the free side part of the fastener.

18. A process for the continuous production of extensible fasteners as claimed in any one of claims 11 to 13 from support bands (4), extensible central parts (2), and additional parts, all these elements being supplied in rolls precoated with adhesive, charaterized by the following stages, taken in combination:

a) the support band precoated with adhesive on both faces is unwound,

b) the support band is precut along a line parallel to the direction of advance by means of a precutting tool of a type known per se,

c) the band of extensible central parts is bonded on the first face of the support band,

d) the additional fastening parts are bonded on the second face of the support band, along a narrow longitudinal band of the said parts,

e) the resulting structure according to d) is cut up into a series of fasteners, which are then fastened on the diaper panties in course of manufacture.

FIG.1

FIG.2

FIG.3

FIG.4

FIG.5

FIG.6

FIG.7

FIG.8